# NOUVEAU FASCICULE DE BREVET EUROPEEN

(11) **EP 1 037 923 B2**
(45) Date de publication et mention de la décision concernant l'opposition: **09.09.2015**
(45) Mention de la délivrance du brevet: 16.03.2005
(21) Numéro de dépôt: 98959975.8
(22) Date de dépôt: 14.12.1998
(51) Int. Cl.: C07K 14/755, A61K 38/37

(54) **PROCEDE DE PREPARATION PAR FILTRATION D'UNE SOLUTION DE FACTEUR VIII SECURISEE VIRALEMENT**
HERSTELLUNGSVERFAHREN EINER VIRUSFREIEN FAKTOR VIII-LÖSUNG DURCH FILTRATION
METHOD FOR PREPARING BY FILTRATION A VIRALLY SECURE FACTOR VIII SOLUTION

(30) Priorité: 15.12.1997 FR 9715888
(43) Date de publication de la demande: 27.09.2000
(73) Titulaire: Laboratoire Français du Fractionnement et des Biotechnologies, 91940 Les Ulis (FR)
(72) Inventeur: CHTOUROU, Abdessatar (Sami), F-78990 Elancourt (FR); NOGRE, Michel, F-92170 Vanves (FR); PORTE, Pierre, F-91250 Saint-Germain-les-Corbeil (FR)
(74) Mandataire: Regimbeau
(86) Numéro de dépôt international: PCT/FR1998/002715
(87) Numéro de publication internationale: WO 1999/031138

(56) Documents cités:
- EP-A- 0 197 554
- EP-A- 0 383 645
- EP-A- 0 468 181
- WO-A-91/18017
- WO-A-96/00237
- WO-A1-96//00237
- WO-A1-98//37086
- US- - 4 758 657
- DJ. JOSIC ET AL.: 'Purification of factor VIII and von Willebrand factor from human plasma by anion-exchange chromatography' JOURNAL OF CHROMATOGRAPHY B vol. 662, 1994, pages 181 - 190
- M. BURNOUF-RADOSEVICH ET AL.: 'Nanofiltration, a New Specific Virus Elimination Method Applied to High-Purity Factor IX and Factor IX and Factor XI Concentrates', vol. 67, 1994, KARGER, BASEL pages 132 - 138
- J.O'GRADY ET AL.: 'Virus Removal Studies Using Nanofiltration Membranes', vol. 88, 1996 pages 319 - 326
- H. MAERZ ET AL.: 'Improved removal of viruslike particles from purified monocional antibody IgM preparation via virus filtration' NATURE BIOTECHNOLOGY vol. 14, 14 Mai 1996, pages 651 - 652
- Rote Liste 1996, 48001 to 48052, Arzneimittelverzeichnis des Bundesverbandes derPharmazeutischen Industrie E.V. (BPI), des Verbandes Forschender Arzneimittelhersteller E.V. (VFA), des Bundesfachverbandes der Arzneimittel-Hersteller E.V. (BAH) und des Verbandes aktiver Pharmaunternehmen E.V. (VAP);

## Description

La présente invention concerne un procédé de préparation par filtration d'une solution de facteur VIII sécurisée viralement et essentiellement dépourvue de facteur von Willebrand (vWF) de haut poids moléculaire, à partir d'une solution de facteur VIII de haute ou très haute pureté et contenant des complexes de haut poids moléculaire facteur VIII-vWF.

Le facteur VIII est un composant protéique du sang utilisé depuis de nombreuses années pour le traitement d'individus souffrant d'hémophilie A, une maladie congénitale causée par une déficience ou une absence de facteur VIII dans le sang. On a utilisé pendant longtemps des concentrés plasmatiques enrichis en facteur VIII pour le traitement des patients.

Les concentrés les plus couramment utilisés étaient le cryoprécipité et les concentrés purifiés obtenus à partir du cryoprécipité. On appelle usuellement cryoprécipité, un précipité obtenu à partir d'un plasma humain congelé par une technique de fractionnement plasmatique à basse température. Le plasma congelé est ramolli à une température d'environ -5°C à -15°C puis réchauffé lentement sous agitation à une température qui ne dépasse pas 3°C. Dans ces conditions, le plasma gelé fond partiellement pour donner une phase liquide et une phase solide, la phase solide étant alors récupérée par centrifugation pour donner le cryoprécipité, qu'il convient alors de purifier pour obtenir des préparations de facteur VIII suffisamment pures. Cette fraction cryoprécipitée est composée essentiellement de fibronogène, fibronectine, facteur VIII et facteur Von Willebrand (vWF).

Dans cette fraction cryoprécipitée, le facteur VIII est généralement associé au vWF qui stabilise le facteur VIII par complexation.

Pendant longtemps, les étapes de purification visèrent essentiellement à séparer du facteur VIII, des protéines indésirables telles que notamment le fibrinogène et la fibronectine.

Toutefois, on sait maintenant qu'un des problèmes essentiels liés à la préparation du facteur VIII à partir du plasma, réside dans la nécessité d'inactiver/d'éliminer des virus originellement contenus dans le sang avec des rendements satisfaisants.

Bien qu'il soit difficile d'établir une liste exhaustive de ces virus, on peut citer en particulier les différents virus hépatiques, hépatite A, hépatite B, hépatite C, hépatite G ou encore, les différentes formes du virus du Sida (VIH).

De nombreuses techniques d'inactivation virale ont ainsi été développées telles que le chauffage à sec, la pasteurisation, le traitement solvant-détergent. L'ensemble de ces techniques est relativement efficace vis à vis des virus enveloppés mais l'inactivation ou l'élimination des virus nus, notamment des petits virus tels que le parvovirus B19 ou le virus de l'hépatite A, reste l'un des problèmes majeurs.

Des technologies plus récentes utilisent les capacités de rétention virale de membranes de faible taille de pores. Ces technologies présentent effectivement une efficacité notable vis à vis de virus de petite taille tels que les parvovirus B19 ou le virus de l'hépatite A et peuvent être appliquées à des protéines de faible poids moléculaire. Cependant, les seuils de coupure utilisés, qui sont inférieurs à 900 kD, ne permettent pas d'envisager la filtration de protéines ou complexes protéiques de haut poids moléculaire comme le facteur VIII sans perte majeure de rendement.

Ainsi par exemple, la publication de brevet WO96/00237 décrit une méthode pour améliorer la filtrabilité des protéines dans une solution contenant au moins une macromolécule, qui consiste à utiliser une solution dans laquelle la teneur totale en sel varie de 0.2 M à saturation de la solution avec le sel concerné. Josic et al., J. Chromatogr. B. Biomed. Appl.; Vol. 662, No. 2, 1994 décrit l'utilisation de Ca²⁺ pour séparer le facteur VIII du facteur vWF. Cette forte teneur en sel a pour effet d'augmenter les rendements de filtration. De préférence, l'étape de filtration est mise en oeuvre à un stade où l'activité spécifique de la macromolécule d'intérêt est déjà très élevée, de sorte qu'il est possible d'utiliser un filtre à structure très fine et d'éliminer des virus très petits.

Cependant, ce document n'envisage absolument pas la filtration de solutions contenant des molécules de très haut poids moléculaire telles que le facteur VIII, et notamment le facteur VIII d'origine plasmatique, lequel est généralement associé à du vWF, formant ainsi des complexes de haut poids moléculaire. Ce document cite l'exemple d'une forme délétée du facteur VIII recombinant avec un poids moléculaire moyen de l'ordre de 170 kD et totalement dépourvu en vWF. Tous les exemples portent sur le facteur IX dont le poids moléculaire moyen est de l'ordre de 55 kD, et ce document souligne que la méthode décrite est particulièrement adaptée à cette taille de molécule, le seuil de coupure du filtre devant par ailleurs être légèrement plus élevé que la taille de la molécule à filtrer. Ainsi, pour le facteur IX, le filtre utilisé a un seuil de coupure de 70 kD.

Or, comme indiqué précédemment, le facteur VIII, selon son origine, peut se présenter sous une forme complexée qui lui donne une taille significative, laquelle peut atteindre 20 000 kD en termes de poids moléculaire et plusieurs dizaines de nanomètres en terme de taille particulaire (> 106 nm pour l'axe principal de la molécule, Eppel et al., Langmuir 1993, 9, 2281-88, American Chemical Society). Aucun moyen n'est disponible actuellement pour filtrer de façon satisfaisante, ce type de molécules et simultanément éliminer des virus de petite taille tels que par exemple les Parvovirus B19 qui ont une taille de l'ordre de 18 à 20 nm.

Les inventeurs ont maintenant trouvé de façon surprenante qu'il était possible d'obtenir par filtration une solution de facteur VIII sécurisée viralement et essentiellement dépourvue de vWF de haut poids moléculaire, à partir d'une solution contenant du facteur VIII de haute ou très haute pureté contenant des complexes de haut poids moléculaire facteur VIII-vWF.

C'est pourquoi l'invention a pour objet un procédé de préparation par filtration d'une solution de facteur VIII sécurisée viralement, selon lequel :
- on prépare une solution de haute ou très haute pureté comprenant du complexe protéique facteur VIII-vWF;
- on met en oeuvre une étape a) permettant la dissociation des complexes de haut poids moléculaire facteur VIII-vWF au moyen d'un ion chaotropique en quantité suffisante pour permettre la dissociation, et l'obtention d'une solution essentiellement dépourvue de facteur VIII associé à du vWF de haut poids moléculaire,
- on met en oeuvre une étape b) de filtration de ladite solution sur un filtre hydrophile ayant une porosité de 15nm.

La préparation par filtration d'une solution de FVIII permet avantageusement d'obtenir une solution essentiellement dépourvue de vWF de haut poids moléculaire, notamment de vWF ayant un degré de polymérisation supérieur ou égal à 15, c'est-à-dire dans laquelle le vWF est contrôlé à la fois qualitativement et quantitativement. Le procédé selon l'invention permet également d'augmenter significativement les rendements de filtration grâce à la diminution de la quantité de complexes protéiques de haut poids moléculaire, et d'obtenir une solution ayant un degré de pureté satisfaisant tout en assurant l'élimination virale des virus pathogènes humains de taille ≥15nm.

La dissociation du complexe FVIII-vWF est obtenue au moyen d'au moins un ion chaotropique présent en quantité suffisante pour permettre la dissociation. Tous les ions connus pour avoir une activité chaotropique sont utilisables.

Il s'agira de préférence d'ions divalents apportés à la solution de facteur VIII sous forme d'une solution saline de 0,2M à saturation des sels.

On peut citer à titre d'exemple non limitatif, CaCl₂, ou encore MgCl₂. On utilisera de préférence une solution de CaCl₂. La concentration de la solution sera de préférence environ 0.35M.

On a constaté que, outre les conditions propres à la filtration, les conditions utilisées pour la dissociation ont une influence sur le rendement de la filtration ultérieure. Les paramètres qui définissent l'ensemble de ces conditions sont, lors de l'étape de dissociation, la nature des sels et leur concentration, et lors de l'étape de filtration, la pression et la température.

De façon surprenante, les rendements de filtration sont considérablement améliorés lorsque la pression transmembranaire lors de la filtration est abaissée à des valeurs très faibles, en deçà des seuils de recommandations préconisés par le fournisseur de filtres.

La température exerce aussi une influence non négligeable sur les rendements de filtration, des valeurs trop fortes ou trop faibles de la température ayant pour effet d'augmenter le nombre des formes multimères du vWF. Avantageusement, on choisira une température de l'ordre de 35 ± 5°C.

Parmi les filtres à virus disponibles sur le marché ou en cours de développement, on peut citer par exemple la membrane Planova® 15N commercialisée par la Société Asahi Chemical Industry. Dans ce cas, le filtre est utilisé de préférence à une pression inférieure à 0,3 bar, avantageusement inférieure à 0,2 bar.

Différentes techniques de filtration peuvent être mises en oeuvre. Les techniques les plus courantes sont les techniques de filtration tangentielle ou de filtration frontale qui peuvent être mises en oeuvre avec les mêmes types de filtres.

La solution de facteur VIII de départ, préalablement purifiée, peut être préparée de différentes façons, par exemple, à partir d'une fraction plasmatique telle que la fraction cryoprécipitée du plasma, ou encore par voie recombinante. Toutes les conditions de préparation connues de l'homme du métier peuvent être utilisées. De manière non limitative, on peut citer les méthodes de purification suivantes permettant l'obtention d'une solution de facteur VIII pré-purifiée utilisable pour la mise en oeuvre du procédé selon l'invention :
. Chromatographie d'échange d'ions selon par exemple l'une des variantes décrites dans les brevets EP-B-359 593, EP 0 343 275, US 4 743 680, WO 97/17370 et EP 0 173 242.
. Chromatographie d'immunoaffinité par exemple selon l'une des variantes décrites dans les publications de demandes de brevet WO 97/39033, EP 0 286 323 ou dans le document Zimmerman et Fulcher, Thrombosis Res., Suppl. VII, p 58, 1987 ; Berntorp et Nilson, Thrombosis Res., Suppl. VII, p 60, 1987.
. Chromatographie de filtration sur gel en milieu dissociant ou non telle que décrite par P.J. Fay (P.J. Fay et al. Proc. Nat. Acad. Sci. USA vol 79 p 7200-7204, 1982).
. Chromatographie d'affinité sur héparine immobilisée telle que décrite dans la publication de demande de brevet WO 93/22337.

Typiquement, la purification par chromatographie d'échange d'ions à partir d'une fraction plasmatique telle que la fraction cryoprécipitée du plasma, comporte une étape d'inactivation virale permettant l'inactivation des virus enveloppés. On peut utiliser différents systèmes chromatographiques, les conditions d'adsorption puis d'élution de la fraction concentrée en facteur VIII pouvant ensuite avoir une influence sur les rendements ultérieurs du procédé. On peut faire varier la nature de la matrice et de l'échangeur d'ions. On peut ainsi mettre en oeuvre un système chromatographique d'échange d'ions faibles, tel que par exemple le gel Toso Haas Toyopearl-DEAE 650 M, ou encore un système chromatographique d'échange d'ions forts, tel que par exemple le gel Q-Sepharose Fast Flow (Pharmacia Biotech). Lorsque la solution de départ est préparée par purification par échange d'ions, elle contient une quantité significative de facteur VIII associé à du vWF de haut poids moléculaire et l'étape a) est indispensable.

La dissociation de l'étape a) peut être effectuée en même temps que l'élution ou, suivant un autre aspect, postérieurement à l'élution. Une élution effectuée en présence d'un sel chaotrope a pour effet d'augmenter le rendement d'élution par rapport à une élution effectuée en présence d'un sel tel que NaCl, tout en assurant la dissociation, indispensable pour réaliser l'étape de filtration ultérieure dans les conditions requises.

Selon un mode de réalisation préféré, la solution concentrée de facteur VIII obtenue à l'issue de la purification par chromatographie d'échange d'ions est donc éluée dans les conditions dissociantes de l'étape a), c'est-à-dire en présence d'un ion chaotropique.

La purification préalable de la solution de facteur VIII de départ peut également être obtenue par une technique de précipitation à l'héparine. Dans ce cas, on adsorbe par exemple, une fraction cryoprécipitée du plasma sur un gel d'hydroxyde d'aluminium, en présence d'héparine, avec un refroidissement à une température d'environ 14°C à environ 19°C et centrifugation. Une première étape d'inactivation virale, mise en oeuvre sur le surnageant de précipitation, peut être effectuée avantageusement par un traitement solvant-détergent tel que décrit dans la publication européenne de brevet EP-A-0313275. Le surnageant de précipitation est alors ajusté en pH et osmolalité avant l'étape de chromatographie par échange d'ions.

Enfin, on peut également envisager de partir d'une solution de facteur VIII recombinant, qui peut nécessiter une étape supplémentaire d'inactivation virale. Il ne sera pas nécessaire de mettre en oeuvre l'étape a).

De préférence, la solution de facteur VIII de départ aura une activité spécifique au moins égale à 50 UI/mg, de préférence au moins égale à 100 UI/mg, la filtrabilité de la solution augmentant avec l'activité spécifique du facteur VIII.

L'activité spécifique telle qu'indiquée s'entend avant ajout éventuel d'albumine en vue de la stabilisation du facteur VIII.

Plus particulièrement, on utilisera une solution de départ dans laquelle la concentration en facteur VIII : C est de environ 2 à environ 100 U/ml de préférence de environ 10 à environ 50 U/ml.

La teneur en protéines de la solution de facteur VIII de départ sera avantageusement de environ 0.05 à environ 0.5 mg/ml, de préférence de environ 0.1 à environ 0.5 mg/ml.

La teneur en protéines est déterminée par la technique de dosage des protéines de Bradford (trousse de dosage commercialisée par la société Pierce).

Une fois la filtration effectuée, les facteurs VIII et von Willebrand qui ont été filtrés, sont réassociés sous forme de complexes, après élimination des agents dissociants, par exemple par dialyse, et on récupère, éventuellement après une étape de lyophylisation, la solution de facteur VIII formulée pour une utilisation commerciale.

L'invention a également pour objet une solution de facteur VIII sécurisée viralement et essentiellement dépourvue de vWF de haut poids moléculaire susceptible d'être obtenue par le procédé selon l'invention.

Enfin, l'invention concerne les solutions obtenues selon l'invention à titre de médicament, plus particulièrement pour traiter l'hémophilie A.

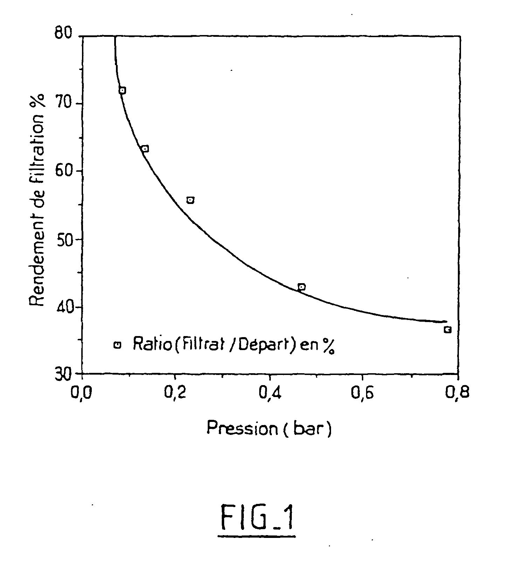

L'invention sera décrite plus en détails à l'aide des exemples qui suivent, qui illustrent l'invention sans toutefois en limiter la portée. Les exemples sont accompagnés de la Figure 1 qui représente :
. Figure 1 : courbe qui met en évidence la variation de la filtrabilité du facteur VIII sur membrane Planova® 15 N en fonction de la pression appliquée au système. En abscisse, figure la pression exprimée en bars et en ordonnées, figure le rendement de filtration exprimé en pourcentage (rapport entre l'activité FVIIIC exprimée en UI/ml du filtrat sur celle du produit de départ).

### EXEMPLE 1 : Préparation d'une solution de facteur VIII par filtration à partir d'une fraction cryoprécipitée du plasma.

La préparation de la solution de facteur VIII de départ est effectuée conformément à l'enseignement du brevet FR 2 632 309 dont le contenu est ici incorporé par référence.

835 g de cryoprécipité représentant 113,5 litres de plasma sont ressolubilisés par agitation dans une solution d'eau héparinée (3UI/ml) à la température ambiante pendant 30 minutes. 4217 ml de solution riche en facteur VIII et en protéines sont clarifiés par adsorption sur 90 g de gel d'hydroxyde d'aluminium et par précipitation acide (pH 6,50) et abaissement de la température entre 15 et 19°C. Un précipité enrichi en fibrinogène et fibronectine est séparé par centrifugation, ce qui permet d'obtenir une solution limpide de facteur VIII de pureté intermédiaire qui est inactivée des virus enveloppés par addition de Polysorbate 80 et de Tri-n-butyl Phosphate (respectivement en solution q.s.p 1% et 0,3%) pendant au moins 6 heures à pH 7,1.

5172 ml de solution de facteur VIII viro-inactivée vis à vis des virus enveloppés sont adsorbés à 560 ml de gel de chromatographie d'échange d'anions faibles (TosoHaas Toyopearl-DEAE 650M) préalablement équilibré en solution saline tamponnée. Après 2 heures d'adsorption, le gel est lavé par une solution saline d'osmolalité 390 mOsm/Kg et tamponnée à pH 7,00. La fraction non adsorbée sur le gel est riche en fibrinogène. Le gel est ensuite élue d'une fraction enrichie en facteur von Willebrand par augmentation de l'osmolalité à 452 mOsm/Kg. La fraction concentrée de très haute pureté en facteur VIII est ensuite éluée par modification du pH à 6,0 et augmentation de la force ionique. La fraction éluée est ensuite ajustée en CaCl₂ à une concentration de 0.35M et une osmolalité de 1300 ± 100 mOsm/Kg. Cette fraction est constituée d'un mélange de facteur VIII et de facteur von willebrand sous forme dissociée grâce à l'action de la forte teneur en calcium.

1260 ml de solution stable à +4°C sont réchauffés extemporanément à +35°C pour subir une étape d'élimination de virus par filtration à l'aide d'un filtre BMM Planova® 15N au seuil de porosité 15 nanomètres et ayant une surface de 0,12 m². Le débit est maintenu au cours de la filtration de telle manière à ce que la pression transmembranaire soit toujours inférieure à 0,2 bar. Après filtration du facteur VIII, 210 ml de solution saline tamponnée d'osmolalité 1300 mOsm/Kg sont ensuite filtrés sur la membrane afin de récupérer 1470 ml de solution de facteur VIII exempte de virus pathogènes. La solution tampon permet d'équilibrer les filtres en osmolalité et pH et sert à rincer les filtres après filtration du facteur VIII. La solution de facteur VIII obtenue est appauvrie en facteur von Willebrand de très haut degré de polymérisation (≥ à 15) mais contient suffisamment de facteur von Willebrand de degré de polymérisation ≥ à 5 et ≥ à 10 pour recomplexer le facteur VIII après dialyse.

### Résultats :

Le Tableau 1 indique aux différentes étapes du procédé selon l'invention les quantités de facteur VIII obtenues ainsi que l'activité spécifique (AS), la teneur en protéines et le rendement de l'étape en cause.

**TABLEAU 1**

| | **Volume (ml)** | **FVIII:C (UI/ml)** | **Protéines mg/ml** | **FVIII total (UI)** | **AS (UI/mg)** | **Rendement (%)** |
|---|---|---|---|---|---|---|
| **Solution éluée de chromatographie** | 1260 | 22 | 0,32 | 27720 | 69 | 100 |
| **Solution de facteur VIII filtrée à 15 nm** | 1470 | 12 | 0,14 | 17640 | 86 | 64 |
| **Solution dialysée et concentrée** | 149 | 108 | 1,13 | 16092 | 95 | 58,1 |

EXEMPLE 2 : Les conditions sont identiques à celles de l'exemple 1 sauf que 10 000g de cryoprécipité représentant 1 330 litres de plasma sont mis en oeuvre. 13 700 ml de solution de facteur VIII viro-inactivée vis à vis des virus enveloppés sont filtrés. Après filtration du facteur VIII, 2 litres de solution tampon, d'osmolalité 1300 mOsm/Kg sont filtrés afin de récupérer 15 700 ml de solution de facteur VIII exempte de virus pathogènes. La membrane de filtration utilisée est une membrane BMM Planova® 15N d'une surface de 1,0 m².

Le Tableau 2 reproduit ci-après, indique pour une filtration d'un équivalent 1330 litres de plasma, sur membrane BMM Planova® 15N d'une surface de 1,0 m², les quantités de facteur VIII obtenues aux différentes étapes du procédé de filtration ainsi que l'activité spécifique et le rendement de l'étape en cause.

**TABLEAU 2**

| | **Volume (ml)** | **FVIII:C (UI/ml)** | **Protéines mg/ml** | **FVIII total (UI)** | **AS (UI/mg)** | **Rendement (%)** |
|---|---|---|---|---|---|---|
| **Solution éluée de chromatographie** | 13700 | 15,0 | 0,09 | 205500 | 167 | 100 |
| **Solution de facteur VIII filtrée à 15 nm** | 15700 | 7,9 | 0,045 | 124030 | 176 | 60,3 |
| **Solution dialysée et concentrée** | 956 | 119 | 0,72 | 113764 | 165 | 55,5 |

EXEMPLE 3 : Préparation d'une solution de facteur VIII par filtration à partir d'une fraction issue d'un cryoprécipité du plasma et pré-purifiée par précipitation à l'héparine.

La préparation de la solution de facteur VIII de départ est effectuée conformément à l'enseignement du brevet US 4 743 680 dont le contenu est incorporé par référence.

678g de cryoprécipité représentant 92,2 litres de plasma sont ressolubilisés par agitation dans une solution d'eau héparinée (3UI/ml) à la température ambiante pendant 30 minutes. 3424 ml de solution riche en facteur VIII et en protéines sont clarifiés comme dans l'Exemple 1.

4200 ml de solution de facteur VIII viro-inactivée vis à vis des virus enveloppés dans les mêmes conditions que l'Exemple 1, sont adsorbés après acidification à pH 6,50 à 300 ml de gel de chromatographie d'échange d'anions forts (Pharmacia Biotech Q-Sepharose Fast Flow) préalablement équilibré en solution saline tamponnée. Après 2 heures 30 minutes d'adsorption, le gel est lavé par une solution saline d'osmolalité 450 mOsm/Kg et tamponnée à pH 6,50. La fraction non adsorbée sur le gel est riche en fibrinogène. Le gel est ensuite élue d'une fraction enrichie en facteur von willebrand par augmentation de l'osmolalité à 581 mOsm/Kg. La fraction concentrée de très haute pureté en facteur VIII est ensuite éluée par modification du pH à 6,0 et augmentation de la force ionique. La fraction éluée est ajustée en CaCl₂ à une concentration de 0.35M et une osmolalité de 1300 ± 100 mOsm/Kg. Cette fraction est constituée d'un mélange de facteur VIII et de facteur von Willebrand sous forme dissociée grâce à l'action de la forte teneur en calcium.

1280 ml de solution stable à +4°C sont réchauffés extemporanément à +35°C pour subir une étape d'élimination de virus par filtration de la même façon que dans l'Exemple 1 sur une membrane Planova 15N au seuil de porosité 15 nanomètres et ayant une surface de 0,12 m². Un volume de 180 ml de solution tampon d'osmolalité 1300 mOsm/Kg est ensuite filtré afin de récupérer 1460 ml de solution de facteur VIII exempte de virus pathogènes. Cette solution de facteur VIII est appauvrie en facteur von Willebrand de très haut degré de polymérisation (≥ 15) mais contient suffisamment de facteur von willebrand de degré de polymérisation ≥ à 5 et ≥ à 10 pour recomplexer le facteur VIII après dialyse.

### Résultats :

Le Tableau 3 reproduit ci-après, indique aux différentes étapes du procédé de filtration, les quantités de facteur VIII obtenues, la quantité totale de protéines ainsi que l'activité spécifique (AS) et le rendement de l'étape en cause.

**TABLEAU 3**

| | **Volume (ml)** | **FVIII:C (UI/ml)** | **FVIII total (UI)** | **Protéines mg/ml** | **AS (UI/mg)** | **Rendement (%)** |
|---|---|---|---|---|---|---|
| **Solution éluée de chromatographie** | 1200 | 19 | 24320 | 0,20 | 95 | 100 |
| **Solution de facteur VIII filtrée à 15 nm** | 1460 | 13 | 18980 | 0,12 | 108 | 78 |
| **Solution dialysée et concentrée** | 164 | 92 | 15088 | 0,95 | 97 | 62 |

Exemple 4 : Variation de la filtrabilité d'une solution de facteur VIII sur une membrane Planova® 15 N en fonction de la nature et de la concentration des sels utilisés pour la dissociation.

### Résultats :

Le Tableau 4 reproduit ci-après, met en évidence les variations du rendement de filtration en fonction de ces différents paramètres, les autres conditions opératoires du procédé demeurant celles définies à l'exemple 1.

**TABLEAU 4**

| **Agent dissociant (M)** | **Rendement de Filtration (%)** |
|---|---|
| **CaCl₂ 0,35 M** | 45 |
| **NaCl 0,35 M** | 10 |
| **NaCl 1,0 M** | 26 |

L'utilisation d'agents dissociants permet d'augmenter de façon significative la filtrabilité du facteur VIII. L'élimination de ces agents par dialyse après filtration induit une réassociation des complexes facteur VIII - facteur von Willebrand. L'analyse des produits obtenus met en évidence une bonne capacité du facteur von Willebrand à fixer le facteur VIII.

Exemple 5 : Variation de la filtrabilité d'une solution de facteur VIII sur une membrane Planova® 15 N en fonction des paramètres de température et pression.

Le Tableau 5 reproduit ci-après, indique, le sel utilisé pour la dissociation et sa concentration demeurant inchangés, le rendement de filtration obtenu en faisant varier la pression et la température. Lorsque la température est abaissée, pour une pression donnée, on observe une diminution significative du rendement de filtration.

La Figure 1 met en outre clairement en évidence que l'abaissement de la pression transmembranaire à des valeurs très faibles permet une amélioration considérable du rendement.

**TABLEAU 5**

| **Agent dissociant** | **Pression (bar)** | **Température (°C)** | **Rendement de filtration (%)** |
|---|---|---|---|
| **CaCl₂ 0,35M** | ≤ 0,10 | 35 ± 2 | 70 |
| **CaCl₂ 0,35M** | ≤ 0,20 | 35 ± 2 | 58 |
| **CaCl₂ 0,35M** | 0,50 | 35 ± 2 | 45 |
| **CaCl₂ 0,35M** | ≤ 0,20 | 20 ± 2 | 42 |

Exemple 6 : Etude de la capacité de rétention virale du système de filtration dans les conditions de filtration de l'exemple 1.

On utilise comme modèle viral le phage ∅ x 174 dont la taille peut être évaluée à 25-30 nm. La membrane et les conditions opératoires sont celles décrites aux Exemples 1 et 2.

### Résultats :

Les résultats contenus dans le Tableau 6 ci-après mettent en évidence une capacité de rétention virale tout à fait satisfaisante.

**TABLEAU 6**

| | **Surface de filtration (m²)** | **FVIII:C (UI/ml)** | **Volume filtré (1/m²)** | **Charge Virale (log)** | **Rétention Virale (log)** |
|---|---|---|---|---|---|
| **Exemple 1** | 0,01 | 15 | 14 | 8,3 | >6,8 |
| **Exemple 2** | 0,01 | 19 | 15 | 7,5 | >7,0 |

### EXEMPLE 7 : Effet du procédé selon l'invention sur la teneur en vWF dans la solution de facteur VIII.

On a comparé la teneur en vWF ainsi que le profil des multimères de vWF sur une solution de facteur VIII telle que décrite à l'exemple 1, avant et après la mise en oeuvre du procédé selon l'invention.

Les résultats obtenus sont reportés au Tableau 7 ci-après.

**TABLEAU 7**

| | **Avant filtration** | **Après filtration** |
|---|---|---|
| **FVIII (UI/ml)** | 17 | 7, 9 |
| **vWF (UI/ml)** | 5,6 | 0,39 |
| **vWF / FVIII** | 0,33 | 0,05 |
| **multimères de WWF ≥ 15** | 12 % | - |
| **multimères de vWF ≥ 10** | 32,4 % | 3,3 % |
| **multimères de vWF ≥ 5** | 71,5 % | 35,6 % |

Ainsi, la mise en oeuvre du procédé selon l'invention permet bien d'obtenir une solution de facteur VIII essentiellement dépourvue de vWF à haut poids moléculaire, notamment essentiellement dépourvue des formules multimériques avec un degré de polymérisation ≥ 15.

## Revendications

1. Procédé de préparation par filtration d'une solution de facteur VIII sécurisée viralement et essentiellement dépourvue de vWF de haut poids moléculaire, selon lequel :
- on prépare une solution de haute ou très haute pureté comprenant du complexe protéique facteur VIII-vWF;
- on met en oeuvre une étape permettant la dissociation des complexes de haut poids moléculaire facteur VIII-vWF au moyen d'un ion chaotropique en quantité suffisante pour permettre la dissociation, et l'obtention d'une solution essentiellement dépourvue de facteur VIII associé à du vWF de haut poids moléculaire,
- on met en oeuvre une étape de filtration de ladite solution sur un filtre hydrophile ayant une porosité de 15nm.

2. Procédé selon la revendication 1, **caractérisé en ce que** ledit ion chaotropique est un ion divalent.

3. Procédé selon la revendication 2, **caractérisé en ce que** ledit ion divalent est l'ion Ca2+.

4. Procédé selon l'une des revendications 1 à 3, **caractérisé en ce que** ledit ion divalent est ajouté sous forme d'une solution saline de 0.2 M å saturation des sels.

5. Procédé selon la revendication 4, **caractérisé en ce que** ladite solution est une solution de CaCl₂.

6. Procédé selon l'une des revendications 4 ou 5, **caractérisé en ce que** ledit ion Ca 2+ est ajouté sous forme d'une solution CaCl₂, 0.35 M à saturation.

7. Procédé selon la revendication 6, **caractérisé en ce que** ledit filtre est une membrane Planova® 15 N utilisée à une pression inférieure à 0,3 bar, de préférence inférieure à 0,2 bar.

8. Procédé selon l'une des revendications 1 à 7, **caractérisé en ce que** l'étape de filtration est effectuée à une température de l'ordre de 35±5 °C.

9. Procédé selon l'une quelconque des revendications 1 à 8, **caractérisé en ce que** la solution de départ est obtenue par purification d'une fraction plasmatique, notamment la fraction cryoprécipitée du plasma, par chromatographie d'échange d'ions.

10. Procédé selon la revendication 9, **caractérisé en ce que** la fraction concentrée de facteur VIII obtenue à l'issue de la purification par chromatographie d'échange d'ions est éludée dans les conditions dissociantes de l'étape de dissociation.

11. Procédé selon l'une des revendications 1 à 10, **caractérisé en ce que** la solution de facteur VIII de départ est obtenue par pré-purification d'une fraction plasmatique, notamment la fraction cryoprécipitée du plasma, par précipitation à l'héparine.

12. Procédé selon l'une quelconque des revendications 1 à 11, **caractérisé en ce que** la solution de facteur VIII de départ est partiellement inactivée viralement par traitement solvant-détergent.

13. Procédé selon l'une des revendications 1 à 12, **caractérisé en ce que** la solution de facteur VIII de départ comprend du facteur VIII immunopurifié.

14. Procédé selon l'une des revendications 1 à 12, **caractérisé en ce que** la solution de facteur VIII de départ comprend du facteur VIII recombinant.

15. Procédé selon l'une des revendications 1 à 12, **caractérisé en ce que** la solution de départ est d'origine plasmatique.

16. Procédé selon l'une des revendication 1 à 15, **caractérisé en ce que** le facteur VIII dans la solution de départ a une activité spécifique au moins égale à 50 UI/mg, de préférence au moins égale A 100 UI/mg.

17. Procédé selon l'une quelconque des revendications 1 à 16, **caractérisé en ce que** la concentration en facteur VIII C de la solution de facteur VIII de départ est de 2 à 100 U/ml, de préférence de 10 à 50 U/ml.

18. Procédé selon l'une des revendications 1 à 17, **caractérisé en ce que** la teneur en protéines de la solution de facteur VIII de départ est de environ 0.05 à environ 0.5 mg/ml, de préférence de environ 0.1 à environ 0.5 mg/ml.

19. Solution de facteur VIII sécurisée viralement susceptible d'être obtenue par le procédé selon l'une quelconque des revendications 1 à 18.

20. Solution selon la revendication 19, à titre de médicament.

21. Solution selon la revendication 20, à titre de médicament pour le traitement de l'hémophilie A.

## Patentansprüche

1. Verfahren zur Herstellung einer Faktor VIII-Lösung, die bezüglich Viren sicher ist und im Wesentlichen frei von vWF mit hohem Molekulargewicht ist, durch Filtration, gemäß welchem:
- man eine Lösung hoher oder sehr hoher Reinheit herstellt, die Faktor VIII-vWF-Proteinkomplex umfasst;
- man einen Schritt durchführt, welcher die Dissoziation von Faktor VIII-vWF-Komplexen mit hohem Molekulargewicht mittels eines chaotropen Ions in einer ausreichenden Menge, um die Dissoziation zu ermöglichen, und den Erhalt einer Lösung gestattet, die im Wesentlichen frei von Faktor VIII ist, der mit vWF mit hohem Molekulargewicht assoziert ist,
- man einen Schritt der Filtration der Lösung auf einem hydrophilen Filter mit einer Porosität von 15 nm durchführt.

2. Verfahren nach Anspruch 1, **dadurch gekennzeichnet, dass** das chaotrope Ion ein zweiwertiges Ion ist.

3. Verfahren nach Anspruch 2, **dadurch gekennzeichnet, dass** das zweiwertige Ion das Ca²⁺-Ion ist.

4. Verfahren nach einem der Ansprüche 1 bis 3, **dadurch gekennzeichnet, dass** das zweiwertige Ion in Form einer Salzlösung von 0,2 M bis zur Sättigung der Salze zugesetzt wird.

5. Verfahren nach Anspruch 4, **dadurch gekennzeichnet, dass** die Lösung eine CaCl₂-Lösung ist.

6. Verfahren nach einem der Ansprüche 4 oder 5, **dadurch gekennzeichnet, dass** das Ca²⁺-Ion in Form einer CaCl₂-Lösung, 0,35 M bis Sättigung, zugesetzt wird.

7. Verfahren nach Anspruch 6, **dadurch gekennzeichnet, dass** der Filter eine Pianova^{®} 15 N-Membran ist, die bei einem Druck unterhalb von 0,3 Bar, bevorzugt unterhalb von 0,2 Bar verwendet wird.

8. Verfahren nach einem der Ansprüche 1 bis 7, **dadurch gekennzeichnet, dass** der Schritt der Filtration bei einer Temperatur in der Größenordnung von 35 ± 5°C bewirkt wird.

9. Verfahren nach irgendeinem der Ansprüche 1 bis 8, **dadurch gekennzeichnet, dass** die Ausgangslösung durch Reinigung einer Plasmafraktion, insbesondere der kryogefällten Fraktion des Plasmas, mittels Ionenaustauschchromatographie erhalten wird.

10. Verfahren nach Anspruch 9, **dadurch gekennzeichnet, dass** die konzentrierte Faktor VIII-Fraktion, die am Ende der Reinigung mittels Ionenaustauschchromatographie erhalten wird, unter den dissoziierenden Bedingungen des Schritts a) eluiert wird.

11. Verfahren nach einem der Ansprüche 1 bis 10, **dadurch gekennzeichnet, dass** die Faktor VIII-Ausgangslösung durch Vorreinigung einer Plasmafraktion, insbesondere einer kryogefällten Fraktion des Plasmas, mittels Heparin-Fällung erhalten wird.

12. Verfahren nach irgendeinem der Ansprüche 1 bis 11, **dadurch gekennzeichnet, dass** die Faktor VIII-Ausgangslösung durch eine Lösungsmittel-Detergens-Behandlung teilweise bezüglich Viren inaktiviert wird.

13. Verfahren nach einem der Ansprüche 1 bis 12, **dadurch gekennzeichnet, dass** die Faktor VIII-Ausgangslösung immungereinigten Faktor VIII umfasst.

14. Verfahren nach einem der Ansprüche 1 bis 12, **dadurch gekennzeichnet, dass** die Faktor VIII-Ausgangslösung rekombinanten Faktor VIII umfasst.

15. Verfahren nach einem der Ansprüche 1 bis 12, **dadurch gekennzeichnet, dass** die Ausgangslösung plasmatischen Ursprungs ist

16. Verfahren nach einem der Ansprüche 1 bis 15, **dadurch gekennzeichnet, dass** der Faktor VIII in der Ausgangslösung eine spezifische Aktivität von mindestens gleich 50 IE/mg, vorzugsweise mindestens gleich 100 IE/mg aufweist.

17. Verfahren nach irgendeinem der Ansprüche 1 bis 16, **dadurch gekennzeichnet, dass** die Konzentration an Faktor VIII C der Faktor VIII-Ausgangslösung 2 bis 100 E/ml, vorzugsweise 10 bis 50 E/ml beträgt.

18. Verfahren nach einem der Ansprüche 1 bis 17, **dadurch gekennzeichnet, dass** der Gehalt an Proteinen der Faktor VIII-Ausgangslösung etwa 0,05 bis etwa 0,5 mg/ml, vorzugsweise etwa 0,1 bis etwa 0,5 mg/ml beträgt.

19. Faktor VIII-Lösung, die bezüglich Viren sicher gemacht worden ist, erhältlich durch das Verfahren nach irgendeinem der Ansprüche 1 bis 18.

20. Lösung nach Anspruch 19 als Medikament.

21. Lösung nach Anspruch 20 als Medikament für die Behandlung von Hämophilie A.

## Claims

1. A method for preparing by filtration a solution of factor VIII that is virally safe and essentially devoid of high molecular forms of vWF, wherein:
- a solution of high or very high purity comprising factor VIII-vWF protein complex is prepared;
- a step allowing dissociating high molecular weight Factor VIII-vWF complexes by means of a chaotropic ion in sufficient amount to allow dissociation and obtaining a solution substantially devoid of factor VIII associated to high molecular weight vWF is carried out;
- a step of filtration of said solution on a hydrophilic filter having a porosity of 15 nm is carried out.

2. The method of claim 1, **characterized in that** said chaotropic ion is a divalent ion.

3. The method of claim 2, **characterized in that** said divalent ion is Ca²⁺ ion.

4. The method according to one of claims 1 to 3, **characterized in that** said divalent ion is added as a saline solution of 0.2 M to salts saturation.

5. The method of claim 4, **characterized in that** said solution is a CaCl₂ solution.

6. The method according to one of claims 4 or 5, **characterized in that** said Ca 2+ ion is added as a CaCl₂ solution, 0.35 M to saturation.

7. The method of claim 6, **characterized in that** said filter is a Planova® 15 N membrane used at a pressure less than 0.3 bar, preferably less than 0.2 bar.

8. The method according to one of claims 1 to 7, **characterized in that** the filtration step is performed at a temperature of about 35 ± 5 °C.

9. The method according to any one of claims 1 to 8, **characterized in that** the starting solution is obtained by purifying a plasma fraction, notably the cryoprecipitated fraction of plasma, by ion exchange chromatography.

10. The method of claim 9, **characterized in that** the concentrated fraction of factor VIII obtained after purification by ion exchange chromatography is eluted in the dissociating conditions of the dissociation step.

11. The method according to one of claims 1 to 10, **characterized in that** the starting factor VIII solution is obtained by pre-purification of a plasma fraction, notably the cryoprecipitated fraction of plasma, by precipitation with heparin.

12. A method according to any one of claims 1 to 11, **characterized in that** the starting factor VIII solution is partially virally inactivated by solvent-detergent treatment.

13. The method according to one of claims 1 to 12, **characterized in that** the starting factor VIII solution comprises immunopurified factor VIII.

14. The method according to one of claims 1 to 12, **characterized in that** the starting factor VIII solution comprises recombinant factor VIII.

15. The method according to one of claims 1 to 12, **characterized in that** the starting solution is of plasmatic origin.

16. The method according to one of claims 1 to 12, **characterized in that** the factor VIII in the starting solution has a specific activity at least equal to 50 IU / mg, preferably at least equal to 100 IU / mg.

17. The method according to any one of claims 1 to 16, **characterized in that** the concentration of factor VIII C of the starting factor VIII solution is from 2 to 100 U / ml, preferably from 10 to 50 U / ml.

18. The method according to one of claims 1 to 17, **characterized in that** the protein content of the starting factor VIII solution is from about 0.05 to about 0.5 mg / ml, preferably from about 0.1 to about 0.5 mg / ml.

19. A virally safe factor VIII solution that can be obtained by the method according to any one of claims 1 to 18.

20. The solution of claim 20, for use as a medicament.

21. The solution of claim 20, for use as a medicament in the treatment of hemophilia A.
